(19) Europäisches Patentamt
European Patent Office
Office européen des brevets

(11) Veröffentlichungsnummer : **0 029 082**
**B1**

(12) # EUROPÄISCHE PATENTSCHRIFT

(45) Veröffentlichungstag der Patentschrift : **20.04.83**

(51) Int. Cl.³ : **C 07 D457/06**, A 61 K 31/48

(21) Anmeldenummer : **80103892.8**

(22) Anmeldetag : **08.07.80**

(54) **1-Benzylierte Ergometrinderivate, Verfahren zu ihrer Herstellung sowie diese enthaltende pharmazeutische Zusammensetzungen.**

(30) Priorität : **14.11.79 DD 216876**

(43) Veröffentlichungstag der Anmeldung : **27.05.81 Patentblatt 81/21**

(45) Bekanntmachung des Hinweises auf die Patenterteilung : **20.04.83 Patentblatt 83/16**

(84) Benannte Vertragsstaaten : **AT CH DE IT LI**

(56) Entgegenhaltungen :
**GB A 982 737**
**GB A 994 486**
**US A 3 218 324**

**Die Akte enthält technische Angaben, die nach dem Eingang der Anmeldung eingereicht wurden und die nicht in dieser Patentschrift enthalten sind.**

(73) Patentinhaber : **VEB Arzneimittelwerk Dresden**
**Wilhelm-Pieck-Strasse 35**
**DDR-8122 Radebeul 1 (DD)**

(72) Erfinder : **Hempel, Roland, Dr.**
**Ernst-Thälmann-Strasse 20**
**D-8101 Mobschatz (DD)**
Erfinder : **Heidenbluth, Karlheinz, Dr.**
**Dr.-Rudolf-Friedrichs-Strasse 27**
**D-8122 Radebeul (DD)**
Erfinder : **Grawert, Werner**
**Freiligrath Strasse 18**
**D-8122 Radebeul (DD)**
Erfinder : **Bartsch, Reni, Dr.**
**Stephensonstrasse 11**
**D-8045 Dresden (DD)**
Erfinder : **Dauth, Christoph, Dr.**
**Scheringer Strasse 37**
**D-8122 Radebeul (DD)**

(74) Vertreter : **Beetz, sen., Richard, Dipl.-Ing. Patentanwälte Dipl.-Ing. R. Beetz sen. Dipl.-Ing. K. Lamprecht, Dr. Ing. R. Beetz jr. et al**
**Rechtsanwalt Dipl.-Phys. Dr. jur. U. Heidrich, Dr.-Ing. W. Timpe Dipl.-Ing. J. Siegfried Priv.-Doz. Dipl.-Chem. Dr. rer. nat. W. Schmitt-Fumian**
**Steinsdorfstrasse 10 D-8000 München 22 (DE)**

**EP 0 029 082 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

1-Benzylierte Ergometrinderivate, Verfahren zu ihrer Herstellung sowie diese enthaltende pharmazeutische Zusammensetzungen

Die Erfindung betrifft neue 1-benzylierte Ergometrinderivate, die als Therapeutika, insbesondere zur Behandlung therapieresistenter Migräneformen, Verwendung finden können, sowie Verfahren zu ihrer Herstellung.

Es ist bekannt, daß neben den nativen Peptidalkaloiden des Mutterkorns auch halbsynthetische Ergolinderivate wie z. B. der N-(D-6-methyl-8-isoergolenyl)-N.N-diethylharnstoff (A. Cerny und M. Semonsky, Collect. Czechoslov. Chem. Commun. *27* (1962) 1585 ; CS-PS 100 832) den für die Migränetherapie wichtigen Serotoninantagonismus zeigen. Es ist weiter bekannt, daß einige, vorzugsweise uterutonisch wirksame Ergoline wie das D-Lysergsäurebutanolamid oder das D-Lysergsäurepropanolamid durch 1-Methylierung in wertvolle Migränetherapeutika mit ausgeprägt serotoninantagonistischer Wirkung übergeführt werden können. (W. Richtzenhain, Ärztliche Praxis *17* (1965) 1738 ; GB-PS 98 27 37).

Aus der GB-PS 98 27 37 sind Lysergsäurederivate bekannt, die in 1-Stellung aralkyliert bzw. benzyliert sind. Die serotoninantagonistische Wirkung dieser Verbindungen ist genannt.

Der GB-PS 99 44 86 sind Lysergsäure- und Dihydrolysergsäurederivate zu entnehmen, die ebenfalls in 1-Stellung Aralkylgruppen bzw. eine Benzylgruppe aufweisen, wobei die serotoninantagonistische Wirkung einiger derartiger Derivate angesprochen ist.

In der US-PS 3 218 324 sind ferner 1-Benzyldihydroergocristine, 1-Benzyldihydroergocornine und 1-Benzyldihydroergokryptine angegeben, die ebenfalls als Serotoninantagonisten wirken und damit z. B. als Migränemittel pharmazeutisch brauchbar sind.

Alle eingeführten Präparate sind jedoch in der Anwendungsbreite teils durch Nebenwirkungen, teils durch unzureichende Wirkung bei bestimmten Migränefällen eingeschränkt. Versuche, die Wirkung von Ergometrinderivaten durch Einführung substituierter Benzylreste in 1-Stellung günstig zu beeinflussen, wurden nicht beschrieben.

Die Erfindung geht von der überraschenden Feststellung aus, daß Ergometrinderivate, die in 1-Stellung substituierte Benzylgruppen aufweisen, eine unerwartete, signifikant stärkere Antiserotoninwirksamkeit aufweisen als entsprechende Derivate mit unsubstituierten Benzylgruppen in 1-Stellung.

Aufgabe der Erfindung ist die Bereitstellung neuer, hochwirksamer Serotoninantagonisten mit niedriger Toxizität, großer Anwendungsbreite und verminderten Nebenwirkungen, die insbesondere zur Behandlung therapieresistenter Migräneformen Anwendung finden können und mit denen durch Beeinflussung des Wirkungsspektrums neue Anwendungsgebiete erschlossen werden können, sowie von Verfahren zu ihrer Herstellung.

Die Aufgabe wird anspruchsgemäß gelöst.

Vorteilhafte Weiterbildungen sind Gegenstand der Unteransprüche.

Die erfindungsgemäßen 1-benzylierten Ergometrinderivate besitzen die allgemeine Formel I

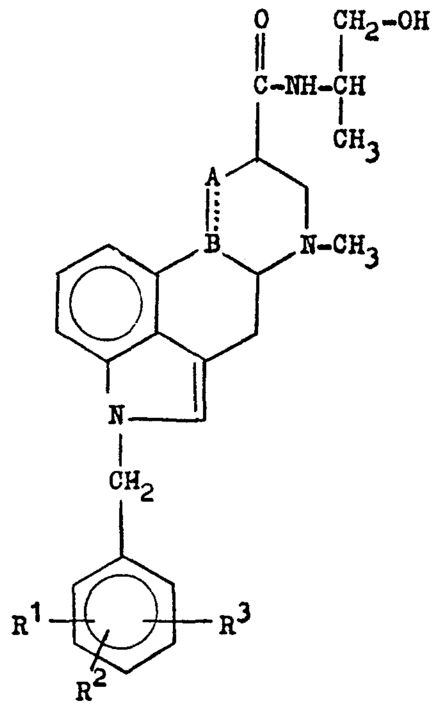

(I)

in der bedeuten :

A $\cdots$ B $-CH=C<$ oder $-CH_2-CH<$

und

$R^1$, $R^2$ und $R^3$ zugleich oder unabhängig H, Halogen, niederes Alkyl, Trifluormethyl oder Alkoxy wobei mindestens einer der Substituenten $R^1$, $R^2$ und $R^3$ nicht gleich H ist.

Die Verbindungen der Formel I können auch in Form physiologisch verträglicher Salze vorliegen, insbesondere in Form der Maleinate, Tartrate und Methansulfonate.

Bevorzugte erfindungsgemäße Verbindungen sind

1-(2'-Fluorbenzyl)-ergometrin,
1-(2'-Fluorbenzyl)-9.10-dihydroergometrin,
1-(3'-Fluorbenzyl)-ergometrin,
1-(3'-Fluorbenzyl)-9.10-dihydroergometrin,
1-(4'-Fluorbenzyl)-ergometrin,
1-(4'-Chlorbenzyl)-ergometrin,
1-(4'-Chlorbenzyl)-9.10-dihydroergometrin,
1-(2'-Brombenzyl)-ergometrin und
1-(2'-Methylbenzyl)-ergometrin.

Das erfindungsgemäße Verfahren zur Herstellung der 1-benzylierten Ergometrinderivate der obigen Formel I ist gekennzeichnet durch Umsetzung eines Ergometrinderivats der allgemeinen Formel II,

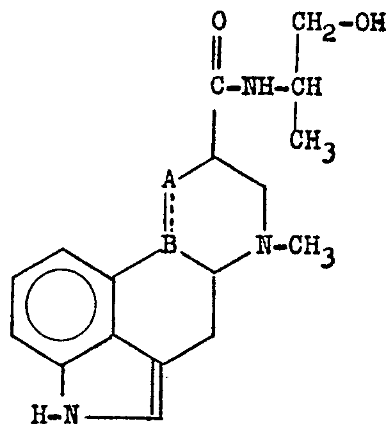

(II)

in der

A $\cdots$ B $-CH=C<$ oder $-CH_2-CH<$

bedeutet, mit einem substituierten Benzylhalogenid der Formel V,

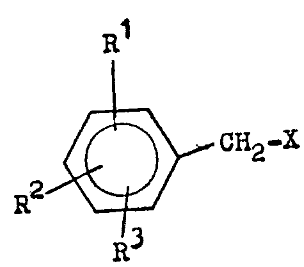

(V)

in der bedeuten :

X Halogen

und

$R^1$, $R^2$ und $R^3$ zugleich oder unabhängig H, Halogen, niederes Alkyl, Trifluormethyl oder Alkoxy, wobei mindestens einer dieser Substituenten nicht gleich H ist, in flüssigem Ammoniak bei dessen Siedetemperatur in Gegenwart von Natriumamid zur entsprechenden Verbindung der Formel I.

Ein weiteres erfindungsgemäßes Verfahren zur Herstellung der 1-benzylierten Ergometrinderivate der Formel I, ist gekennzeichnet durch

a) Umsetzung einer Lysergsäure der allgemeinen Formel III,

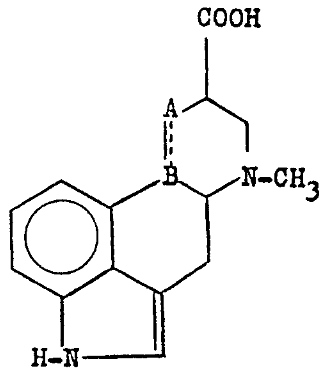

(III)

in der
A ⋯ B $-CH=C<$ oder $-CH_2-CH<$
bedeuten, mit einem substituierten Benzylhalogenid der Formel

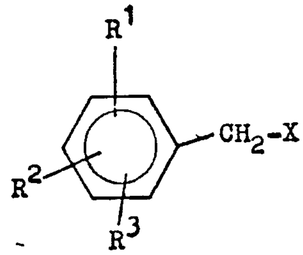

(V)

in der bedeuten :
X Halogen
und
$R^1$, $R^2$ und $R^3$ zugleich oder unabhängig H, Halogen, niederes Alkyl, Trifluormethyl oder Alkoxy, wobei mindestens einer dieser Substituenten nicht gleich H ist, in flüssigem Ammoniak bei dessen Siedetemperatur in Gegenwart von Natriumamid zu einer Verbindung der Formel IV

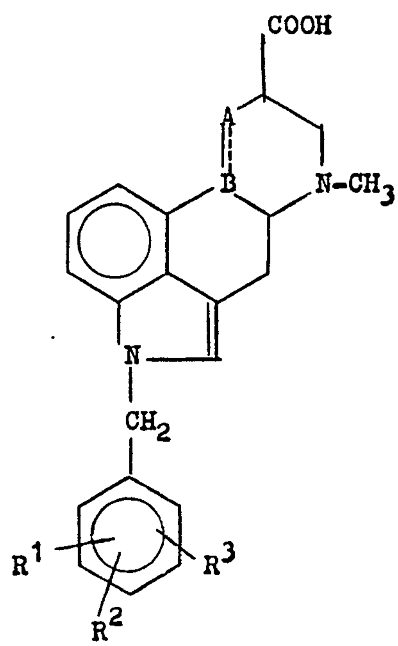

(IV)

mit A ⋯ B und $R^1$, $R^2$ und $R^3$ wie oben und

b) Umsetzung der Verbindung der Formel IV in aktivierter Form mit L-(+)-2-Aminopropan-1-ol zur entsprechenden Verbindung der Formel I.

Wenn Verbindungen der Formel I hergestellt werden sollen, in der die Gruppierung A ⋯ B — $CH_2-CH<$ bedeutet, kann ein Reaktionsprodukt der Formel I mit A ⋯ B gleich $-CH=C<$ durch katalytische

Hydrierung an Raney-Nickel in das entsprechende 9.10-Dihydroderivat übergeführt werden.

Die Reduktion (9.10-Hydrierung) erfolgt katalytisch mit Raney-Ni in niederen Alkoholen oder Dioxan bei 70 °C und 4,9 MPa (50 atü) Wasserstoffdruck.

Die erste der beiden oben erläuterten prinzipiellen Verfahrensweisen beruht auf der an sich bekannten 1-Alkylierung von Verbindungen der Formel II, die auf entsprechende kernsubstituierte Benzylhalogenide der Formel V übertragen wurde.

Da bei dieser Umsetzung eine Reihe von Nebenprodukten entstehen, vorzugsweise die 8-alkylierten Derivate (Troxler, F. und Hofmann, A., Helv. Chim. Acta *40* (1957) 1721 — 32 ; Hempel, R., Grawert, W. und Dauth Ch., Pharmazie *34* (1979) H 5/6, S. 357), ist zur Erzielung hoher Ausbeuten die Einhaltung eines bestimmten Molverhältnisses der Reaktionspartner erforderlich. Für die Benzylierung wurde ein Molverhältnis Ergolinbase : $NaNH_2$ : Benzylhalogenid von 1 : 1,4 : 1,2 als optimal ermittelt. Für die Umsetzung von Lysergsäure und 9.10-Dihydrolysergsäure ist es günstiger, ein Molverhältnis Lysergsäure bzw. 9.10-Dihydrolysergsäure : $NaNH_2$ : Benzylhalogenid von 1 : 3 : 4 anzuwenden. Praktisch wird die Reaktion so ausgeführt, daß man durch Eintragen von Natrium in flüssigen Ammoniak in Gegenwart katalytischer Mengen Eisen (III) nitrat eine Natriumamidlösung herstellt, die Ergolinbase der Formel II einträgt und anschließend die Lösung des substituierten Benzylhalogenids in wenig Äther rasch zufließen läßt. Man rührt noch 30 min bei der Siedetemperatur des flüssigen Ammoniaks und destilliert anschließend das Lösungsmittel ab. Der trockene Rückstand wird in wässeriger Weinsäure aufgenommen und die Lösung durch Ausschütteln mit Ether gereinigt. Nach dem Alkalischmachen wird die Base des Endprodukts abgesaugt bzw. durch Extraktion gewonnen und in ein physiologisch verträgliches Salz übergeführt.

Bei der zweiten oben erläuterten Verfahrensweise wird anstelle der Alkanolamide der Formel II ein Lysergsäurederivat der Formel III aralkyliert ; die erhaltenen Derivate der Formel IV werden durch Abstumpfen der weinsauren Lösung auf pH 5,5 bis 7,0 isoliert, nach einem der in der Peptidchemie üblichen Verfahren beispielsweise durch Umsetzung mit $DMF/SO_3$ in eine aktivierte Form überführt und dann mit Aminopropanol in DMF verknüpft.

Ein weiteres Verfahren zur Herstellung der Verbindungen I, bei denen A$\overset{\cdots}{-}$B für die Gruppierung $-CH_2-CH<$ steht, besteht in der Umsetzung von II mit überschüssigem Alkalihydroxid und V vorzugsweise in einem dipolaren aprotischen Lösungsmittel.

So können z. B. 1-benzylierte 9.10-Dihydroergometrine vorteilhaft durch Umsetzung mit einem entsprechenden Benzylhalogenid und Alkalihydroxid in Dimethylformamid erhalten werden, wobei die technologisch aufwendige Ammoniakverflüssigung entfällt.

Die erfindungsgemäß hergestellten Derivate der Formel I und ihre Salze mit physiologisch verträglichen Säuren besitzen einige wertvolle pharmakologische Eigenschaften, wie beispielsweise aus nachstehenden Tabelle hervorgeht.

Tabelle

Durchschnittliche Antiserotoninwirkung am Serotoninödem an der Ratte als prozentuale Hemmung zur Kontrollgruppe berechnet*

Dosis : 0,005 mg/kg i. p., Werte statistisch gesichert für die Verbindungen A und B

| Substanz | Anzahl der Tiere | prozentuale Hemmung nach Serotoningabe (%) nach 30 min | 90 min | 150 min | 210 min | 270 min |
|---|---|---|---|---|---|---|
| 1-(3'-Fluor-benzyl)-ergometrindimaleinat (Verb. A) | 10 | 44 | 50 | 36 | 55 | 56 |
| 1-(4'-Chlorbenzyl)-ergometrindimaleinat (Verb. B) | 10 | 44 | 45 | 46 | 61 | 58 |
| Methysergid | 10 | 68 | 60 | 36 | 34 | 46 |

* Versuchsanordnung nach Winter, C. H. Risley, E. A. und Nuss, G. W., Proc. Soc., Exp. Biol. Med. *111* (1962) 54.

Die Erfindung wird im folgenden anhand von Ausführungsbeispielen näher erläutert.

Beispiel 1 : 1-(2'-Fluorbenzyl)-ergometrindimaleinat

In einem 1,5 1-Sulfierkolben mit Rührer, Tropftrichter, Trockeneiskühltasche mit Ableitungsrohr werden nach Füllen der Kühltasche mit Aceton/Trockeneis 400 ml Ammoniak verflüssigt. Dazu gibt man 65 mg Eisen (III) nitrat und anschließend portionsweise 395 mg Na in dem Maße, wie die auftretende

Blaufärbung wieder verschwindet. Darauf trägt man 4,0 g trockene Ergometrinbase ein, rührt 20 min und läßt eine Lösung von 2,82 g 2-Fluorbenzylbromid in 5 ml trockenem Ether rasch zufließen. Man rührt noch 30 min unter Rückfluß und arbeitet dann wie folgt auf :

Nach völligem Abdestillieren des Ammoniaks wird der trockene Rückstand in 250 ml 3 %iger wässeriger Weinsäure gelöst, die Lösung zweimal mit je 100 ml Ether gewaschen und die wässerige Phase abgetrennt. Man unterschichtet mit 150 ml Chloroform, stellt mit Ammoniakwasser auf pH 9 ein und wiederholt nach Phasentrennung die Chloroformextraktion. Der Gesamtextrakt wird mit Wasser gewaschen, über $Na_2SO_4$ getrocknet und im Vakuumrotationsverdampfer zur Trockne eingedampft. Darauf löst man die Rohbase (5,22 g) in 15 ml Methanol und fällt mit etherischer Maleinsäurelösung das Dimaleinat aus. Man erhält 5,17 g 1-(2'-Fluorbenzyl)-ergometrindimaleinat.

$C_{26}H_{28}FN_3O_2 \cdot C_4H_4O_4$ (M = 549,6) ;
F. 167,5 °C ; $[\alpha]_D^{20}$ = 3,1° (c = 0,2, Methanol)
In analoger Weise wurden erhalten :

1-(3'-Fluorbenzyl)-ergometrindimaleinat, F. 184,6 °C,
$[\alpha]_D^{20}$ = + 4,2° (c = 0,2, Methanol) ;
1-(4'-Fluorbenzyl)-ergometrindimaleinat, F. 170,0 °C ;
1-(2'-Brombenzyl)-ergometrindimaleinat, F. 192,0 °C,
$[\alpha]_{546}^{20}$ = + 6,1° (c = 0,4, Pyridin) ;
1-(2'-Methylbenzyl)-ergometrindimaleinat, F. 184,5 °C,
$[\alpha]_D^{20}$ = + 4,6° (c = 0,2, Methanol).

Beispiel 2 : 1-(4'-Chlorbenzyl)-ergometrindimaleinat

4,0 g Ergometrinbase werden mit 2,38 g 4-Chlorbenzylchlorid analog Beispiel 1 umgesetzt. Man erhält 4,7 g Rohbase und daraus 4,45 g 1-(4'-Chlorbenzyl)-ergometrindimaleinat.

$C_{26}H_{28}ClN_3O_2$ (M = 566,04) ; F. 175,0 °C,
$[\alpha]_D^{20}$ = + 2,3° (c = 0,2, Methanol).

Beispiel 3 : 1-(2'-Fluorbenzyl)-9.10-dihydroergometrin

Aus 300 ml flüssigem Ammoniak, 80 mg Eisen (III)-nitrat und 630 mg Na wird wie in Beispiel 1 eine Natriumamidlösung hergestellt. Dazu gibt man 5,14 g 9.10-Dihydroergometrinbase und nach 15 min eine Lösung von 5,78 g 2-Fluorbenzylbromid in 10 ml trockenem Ether. Darauf wird noch 30 min gerührt und wie in Beispiel 1 aufgearbeitet. Durch Versetzen der mit Aktivkohle gereinigten methanolischen Lösung mit etherischer Weinsäure erhält man das 1-(2'-Fluorbenzyl)-9.10-dihydroergometrin als Ditartrat.

$C_{26}H_{30}FN_3O_2 \cdot C_4H_6O_6$ (M = 585,6) ;
F. 126,0 °C ; $[\alpha]_D^{20}$ = + 2,5° (c = 0,2, Methanol).

Beispiel 4 : 1-(3'-Fluorbenzyl)-9.10-dihydroergometrinditartrat

5,0 g des gemäß Beispiel 1 hergestellten 1-(3'-Fluorbenzyl)-ergometrins werden in Form der Base in 200 ml Methanol gelöst, mit 4 g Raney-Nickel als Katalysator versetzt und 2 h bei 70 °C und 4,9 MPa (50 atü) hydriert. Nach Absaugen des Katalysators wird die Lösung im Vakuum zur Trockne eingedampft und die Rohbase mit etherischer Weinsäure in das Ditartrat übergeführt. Man erhält 4,3 g 1-(3'-Fluorbenzyl)-9.10-dihydroergometrinditartrat.

$C_{26}H_{30}FN_3O_2 \cdot C_4H_6O_6$ (M = 585,6) ;
F. 124,0 °C ; $[\alpha]_D^{20}$ = – 37,8° (c = 0,2, Methanol)

Beispiel 5 : 1-(4'-Chlorbenzyl)-d-lysergsäure

In einer Apparatur wie in Beispiel 1 beschrieben werden zu 400 mg verflüssigtem Ammoniak 360 mg $Fe(NO_3)_3 \cdot 9H_2O$, 2,4 g Natrium, 9,4 g Lysergsäure sowie 29 g p-Chlorbenzylbromid gegeben.

Der nach Abdunsten des Ammoniaks verbleibende Rückstand wird zwischen 500 ml Ether und 500 ml Wasser verteilt. Nach Filtration und Phasentrennung wird die wässerige Phase auf etwa 1/3 des Ausgangsvolumens konzentriert und mit 20 %iger Schwefelsäure bis pH 1 als Sulfat ausgefällt.

Der erhaltene Niederschlag wird abgetrennt, mit wenig Wasser gewaschen und 5 h bei 80 °C getrocknet. Man erhält 12,5 g eines hellbraunen Kristallpulvers. Die Substanz wird in wässerigem Alkali gelöst, wie üblich mit Aktivkohle gereinigt und erneut als Sulfat ausgefällt. Die so vorgereinigte Substanz wird in wässerig-alkalischer Lösung an der 20-fachen Menge Sephadex ®G 15 chromatographiert. Aus dem Filtrat fällt man die 1-(4'-Chlorbenzyl)-d-lysergsäure bei pH 5,5 bis 6,0 mit 10 %iger Essigsäure aus.

$C_{23}H_{21}ClN_2O_2$ (M = 392,6) ;
F. 136 bis 140 °C.
Nach Umkristallisation mit Aceton :
F. 144 bis 146 °C.

Sulfat $C_{23}H_{21}ClN_2O_2 \cdot H_2SO_4$ (M = 490,6) : F. 171 °C.
Auf analoge Weise wurden z. B. erhalten :
1-(4'-Fluorbenzyl)-d-lysergsäure ; $C_{23}H_{21}FN_2O_2$ ;
F. als Sulfat : 178 °C ;
1-(4'-Chlorbenzyl)-9.10-dihydrolysergsäure ;
$C_{23}H_{23}ClN_2O_2$
F. als Sulfat : 209 °C ; $[\alpha]_D^{20}$ = – 45,3° (c = 0,2, Methanol).

Beispiel 6 : 1-(4'-Chlorbenzyl)-ergometrindimaleinat

Eine Lösung von 5,14 g im Vakuum getrockneter 1-(4'-Chlorbenzyl)-d-lysergsäure und 310 mg getrocknetes Lithiumhydroxid in 50 ml Methanol wird im Vakuum bis zur beginnenden. Abscheidung des Lithiumsalzes eingeengt. Man versetzt das Konzentrat sofort mit 200 ml wasserfreiem Dimethylformamid und dampft im Ölpumpenvakuum bei ca 40 °C Badtemperatur bis auf 60 ml ein. Darauf gibt man unter Rühren und Eiskühlung 26 ml einer 1 M Lösung von DMF · $SO_3$-Komplex zu und gießt diese Mischung nach 20 min auf 6,2 ml (+)-2-Aminopropan-1-ol. Nach weiteren 20 min Rühren versetzt man mit 340 ml dest. Wasser und nach 10 min mit 340 ml 20 %iger wässeriger Weinsäure. Die Mischung wird zunächst dreimal mit 100 ml Chloroform und nach anschließendem Alkalischmachen mit konz. Ammoniaklösung (pH 9 bis 10) nochmals dreimal mit je 200 ml Essigester extrahiert. Die Essigesterextrakte werden vereinigt, mit Wasser gewaschen und nach Trocknung über $Na_2SO_4$ im Vakuum zu einem Sirup eingedampft. Der Rückstand wird in der gerade notwendigen Menge Methanol gelöst und mit einer Lösung von 2,5 g Maleinsäure in 5 ml Methanol bis ph 4 versetzt. Das so erhaltene Rohprodukt (3,1 g) wird durch Umkristallisation mit Methanol/Aktivkohle gereinigt. Man erhält 2,9 g 1-(4'-Chlorbenzyl)-ergometrindimaleinat.
$C_{26}H_{28}ClN_3O_2 \cdot C_4H_4O_4$ (M = 566,04) ;
F. 175 °C.
Auf analoge Weise wurde z. B. erhalten :
1-(4'-Chlorbenzyl)-9.10-dihydroergometrinditartrat, $C_{26}H_{30}ClN_3O_2 \cdot C_4H_6O_6$, (M = 601,8) ;
$[\alpha]_D^{20}$ Base = – 68,8° (c = 0,4, Methanol),
F. 124 °C aus 1-(4'-Chlorbenzyl)-9.10-dihydrolysergsäure (vgl. Beispiel 5).

Beispiel 7 : 1-(4'-Chlorbenzyl)-9.10-dihydroergometrinditartrat

Eine Mischung von 1 g 9.10-Dihydroergometrin und 0,65 g gemörsertes NaOH in 65 ml Dimethylformamid wird 30 min bei Raumtemperatur gerührt. Unter weiterem Rühren werden 1,4 g p-Chlorbenzylbromid, gelöst in 10 ml Dimethylformamid, zugetropft. Die Aufarbeitung kann nach zwei Verfahrensvarianten geschehen :

Variante A

Das Reaktionsgemisch wird im Vakuumrotationsverdampfer bei 40 °C Badtemperatur im Ölpumpenvakuum zur Trockne eingedampft. Der erhaltene Rückstand (ca 1,9 g) wird in der notwendigen Menge $CHCl_3$ aufgenommen und an der 50-fachen Menge $Al_2O_3$ (Aktivitätsstufe 1, neutral) mit Chloroform als Elutionsmittel chromatographiert. Nach der Elution von Nebenprodukten, die keine Blaufärbung mit Secale-Reagens ergeben, wird das 1-(4'-Chlorbenzyl)-9.10-dihydroergometrin ins Filtrat gewaschen.
F. 201 °C. (Nadeln, aus Methanol).
Der Mischschmelzpunkt mit der nach Beispiel 6 gewonnenen Base zeigte keine Depression.

Variante B

Das Reaktionsgemisch wird im Vakuumrotationsverdampfer bei 40 °C Badtemperatur im Ölpumpenvakuum auf ca. 1/3 des Ausgangsvolumens konzentriert und danach mit 20 %iger Weinsäure bis pH 4 versetzt. Anschließend wird dreimal mit dem gleichen Volumen Chloroform extrahiert und nach Alkalischmachen mit konz. Ammoniaklösung (pH 9 bis 10) wie oben dreimal mit Chloroform extrahiert. Die organischen Extrakte der wässerig-alkalischen Phase werden vereinigt, mit Wasser gewaschen, über Natriumsulfat getrocknet und im Vakuumrotationsverdampfer bei 35 °C Badtemperatur zur Trockne eingedampft. Der Rückstand wird nach Trocknung in Aceton gelöst und mit der berechneten Menge fester Weinsäure versetzt. Nach 12 h Stehen bei 3 °C wird abgesaugt und der Niederschlag mit Ether gewaschen. Umkristallisation aus Aceton/Ether liefert 1-(4'-Chlorbenzyl)-9.10-dihydroergometrinditartrat ; F. 124 °C.
$[\alpha]_D^{20}$ = – 38,1° (c = 1, Methanol) (vgl. Beisp. 6).

Beispiel 8 : Herstellung von Dragees

Zu einer Lösung von 1,76 g Aminoessigsäure, 0,88 g Thioharnstoff und 0,86 g Natriumedetat in

50 ml Wasser gibt man 1,2 110 %ige Gelatinelösung. Mit diesem Granuliermittelgemisch werden 4, 200 kg Lactose, 2,600 kg Kartoffelstärke und 0,140 kg 1-(3'-Fluorbenzyl)-ergometrindimaleinat in der Wirbelschicht granuliert und bei 60 bis 70 °C getrocknet. Nach dem Zumischen von 0,140 kg Talk und 0, 070 kg Magnesiumstearat wird durch ein 0,8-mm-Sieb gesiebt. Das fertige Granulat (ca 7,0 kg) wird zu Drageekernen von 5 mm Durchmesser und 50 mg Bruttomasse verpreßt. Die Kerne werden anschließend nach einem üblichen Verfahren dragiert oder lackiert.

Beispiel 9 : Herstellung einer Injektionslösung

Man löst 0,20 g Aminoessigsäure und 0,50 g Thioharnstoff in etwa 800 ml Wasser zur ad inj., das vorher mit Kohlendioxid begast wurde. In diese Lösung werden 0,20 g 1-(4'-Chlorbenzyl)-ergometrindimaleinat eingetragen und innerhalb von etwa 1 h gelöst. Getrennt davon löst man 8,50 g Natriumchlorid in etwa 100 ml ebenfalls $CO_2$-begastem Wasser ad inj. und mischt diese Lösung dem Ansätz zu. Danach wird mit begastem Wasser ad inj. schließlich auf 1 000,0 ml aufgefüllt und gut gemischt. Der fertige Ansatz wird 15 min lang mit Kohlendioxid nachbegast. Nach der Filtration durch ein geeignetes sterilisiertes Membranfilter wird die Lösung unter sterilen Bedingungen zu 1,1 ml in sterilisierte Ampullen abgefüllt.

## Ansprüche

1. 1-Benzylierte Ergometrinderivate der allgemeinen Formel I,

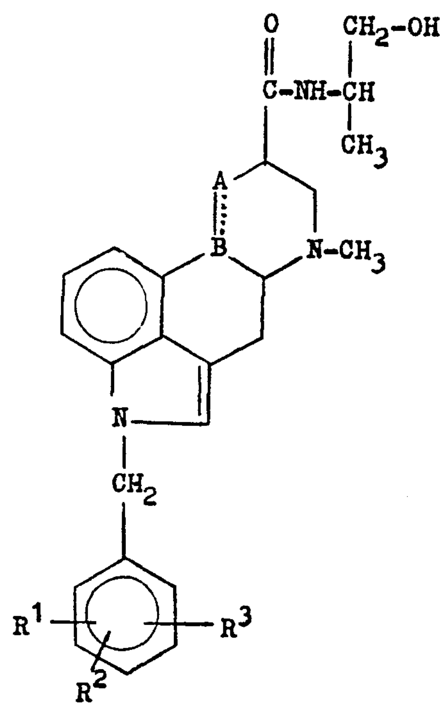

(I)

in der bedeuten :

A ⋯ B —CH = C< oder —$CH_2$—CH<

und

$R^1$, $R^2$ und $R^3$ zugleich oder unabhängig H, Halogen, niederes Alkyl, Trifluormethyl oder Alkoxy wobei mindestens einer der Substituenten $R^1$, $R^2$ und $R^3$ nicht gleich H ist, und ihre Salze mit physiologisch verträglichen Säuren.

2. 1-Benzylierte Ergometrinderivate der Formel I nach Anspruch 1 in Form der Maleinate, Tartrate oder Methansulfonate.

3. 1-(2'-Fluorbenzyl)-ergometrin.

4. 1-(2'-Fluorbenzyl)-9.10-dihydroergometrin.

5. 1-(3'-Fluorbenzyl)-ergometrin.

6. 1-(3'-Fluorbenzyl)-9.10-dihydroergometrin.

7. 1-(4'-Fluorbenzyl)-ergometrin.

8. 1-(4'-Chlorbenzyl)-ergometrin.

9. 1-(4'-Chlorbenzyl)-9.10-dihydroergometrin.

10. 1-(2'-Brombenzyl)-ergometrin.

11. 1-(2'-Methylbenzyl)-ergometrin.

12. Verfahren zur Herstellung der 1-benzylierten Ergometrinderivate der Formel I nach einem der Ansprüche 1 bis 11, gekennzeichnet durch Umsetzung eines Ergometrinderivats der allgemeinen Formel II,

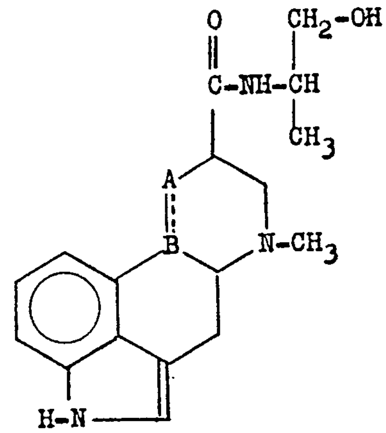

(II)

in der

A ⋯ B $-CH = C<$ oder $-CH_2-CH<$

bedeutet, mit einem substituierten. Benzylhalogenid der Formel V,

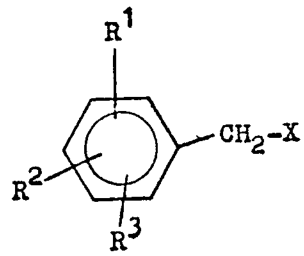

(V)

in der bedeuten :

X Halogen

und

$R^1$, $R^2$ und $R^3$ zugleich oder unabhängig H, Halogen, niederes Alkyl, Trifluormethyl oder Alkoxy, wobei mindestens einer dieser Substituenten nicht gleich H ist in flüssigem Ammoniak bei dessen Siedetemperatur in Gegenwart von Natriumamid zur entsprechenden Verbindung der Formel I.

13. Verfahren zur Herstellung der 1-benzylierten Ergometrinderivate der Formel I nach einem der Ansprüche 1 bis 11, gekennzeichnet durch

a) Umsetzung einer Lysergsäure der allgemeinen Formel III

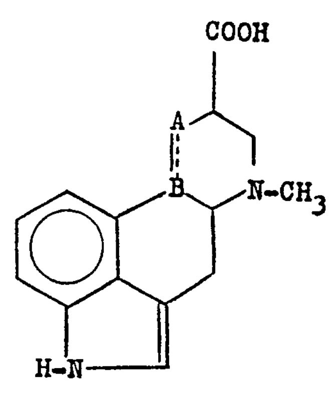

(III)

in der
A ⎓ B —CH = C< oder —$CH_2$—CH<
bedeuten, mit einem substituierten Benzylhalogenid der Formel

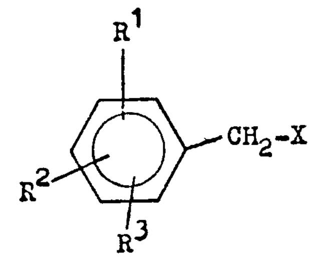

(V)

in der bedeuten :
X Halogen
und
$R^1$, $R^2$ und $R^3$ zugleich oder unabhängig H, Halogen, niederes Alkyl, Trifluormethyl oder Alkoxy, wobei mindestens einer dieser Substituenten nicht gleich H ist, in flüssigem Ammoniak bei dessen Siedetemperatur in Gegenwart von Natriumamid zu einer Verbindung der Formel IV

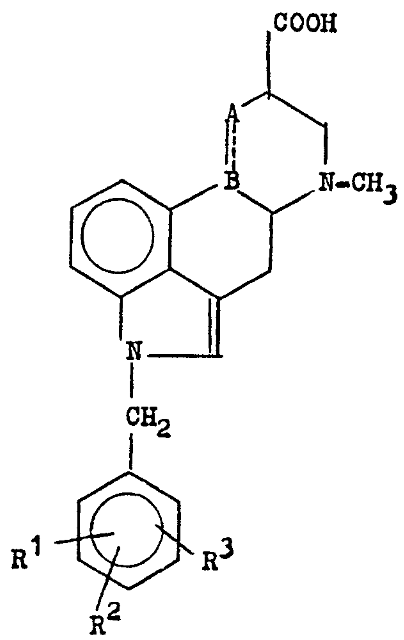

(IV)

mit A ⎓ B und $R^1$, $R^2$ und $R^3$ wie oben und

b) Umsetzung der Verbindung der Formel IV in aktivierter Form mit 1-(+)-2-Aminopropan-1-ol zur entsprechenden Verbindung der Formel I.

14. Verfahren nach Anspruch 12 oder 13, dadurch gekennzeichnet, daß Reaktionsprodukte der Formel I mit A ⎓ B gleich —CH = C< durch katalytische Hydrierung an Raney-Nickel in das entsprechende 9.10-Dihydroderivat übergeführt werden.

15. Verfahren nach Anspruch 13, dadurch gekennzeichnet, daß in Schritt (b) als aktivierte Form der Verbindung der Formel IV das gemischte Anhydrid mit Schwefelsäure verwendet wird.

16. Verfahren nach Anspruch 12 zur Herstellung von Verbindungen der Formel I mit A ⎓ B gleich —$CH_2$—CH<, dadurch gekennzeichnet, daß eine Lösung der Verbindung der Formel V in Dimethylformamid bei Raumtemperatur zu einem Gemisch aus einer Verbindung der Formel II mit A ⎓ B wie oben und einem gepulverten Alkalihydroxid in Dimethylformamid zugetropft wird.

17. Verfahren nach einem der Ansprüche 12 bis 16, dadurch gekennzeichnet, daß die erhaltenen Basen mit physiologisch verträglichen Säuren in die entsprechenden Salze übergeführt werden.

18. Verfahren nach Anspruch 17, dadurch gekennzeichnet, daß die Maleinate, Tartrate oder Methansulfonate hergestellt werden.

19. Pharmazeutische Zusammensetzungen gekennzeichnet durch ein 1-benzyliertes Ergometrinderivat der Formel I nach einem der Ansprüche 1 bis 11 bzw. eines ihrer physiologisch verträglichen Säureadditionssalze als Wirkstoff.

20. Migränetherapeutika gemäss Anspruch 19 in Form von Tabletten, Dragees, Tropflösungen oder Injektionslösungen mit einem Wirkstoffgehalt von 0,25 bis 2,0 mg pro Dosiereinheit.

## Claims

1. 1-Benzylated ergometrinederivatives of the general formula I

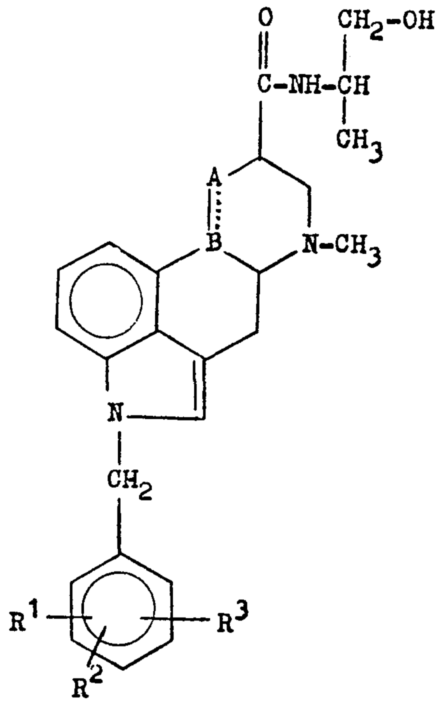

(I)

wherein

A ⋯ B is $-CH=C<$ or $-CH_2-CH<$

and

$R^1$, $R^2$ and $R^3$ are simultaneously or independently H, halogen, lower alkyl, trifluoromethyl or alkoxy, at least one of the substituents $R^1$, $R^2$ and $R^3$ not being H, and their salts with physiologically acceptable acids.

2. 1-Benzylated ergometrine derivatives of the formula I according to claim 1 in the form of the maleinates, tartrates or methane-sulfonates.

3. 1-(2'-fluorobenzyl)-ergometrine.

4. 1-(2'-fluorobenzyl)-9.10-dihydroergometrine.

5. 1-(3'-fluorobenzyl)-ergometrine.

6. 1-(3'-fluorobenzyl)-9.10-dihydroergometrine.

7. 1-(4'fluorobenzyl)-ergometrine.

8. 1-(4'-chlorobenzyl)-ergometrine.

9. 1-(4'-chlorobenzyl)-9.10-dihydroergometrine.

10. 1-(2'-bromobenzyl)-ergometrine.

11. 1-(2'methylbenzyl)-ergometrine.

12. A Process for preparing the 1-benzylated ergometrine derivatives of the formula I according to one of Claims 1 to 11, characterized by reaction of an ergometrine derivative of the general formula II

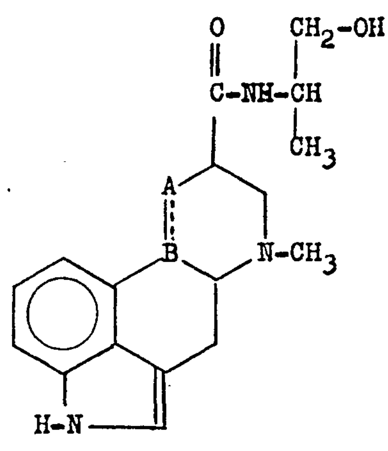

(II)

wherein

A ⋯ B is —CH = C< or $—CH_2—CH<$ .

with a substituted benzylhalogenide of the formula V

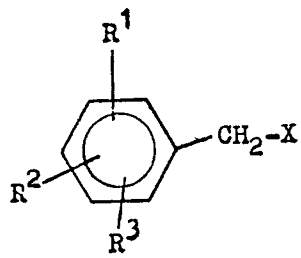

(V)

wherein

X is halogen

and

$R^1$, $R^2$ and $R^3$ are simultaneously or independently H, halogen, lower alkyl, trifluoromethyl or alkoxy, at least one of these substituents not being H, in liquid ammonia at its boiling temperature in the presence of sodium amide to obtain the corresponding compound of the formula I.

13. A process for preparing the 1-benzylated ergometrine derivatives of the formula I according to one of claims 1 to 11, characterized by

a) reaction of a lysergic acid of the general formula III

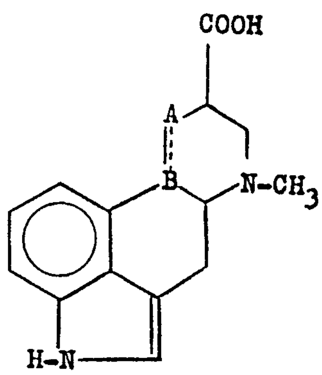

(III)

wherein

A ⋯ B is —CH = C< or $—CH_2—CH<$

with a substituted benzylhalogenide of the formula

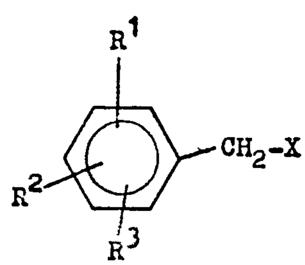

(V)

wherein

X is halogen

and

$R^1$, $R^2$ and $R^3$ are simultaneously or independently H, halogen, lower alkyl, trifluoromethyl or alkoxy, at least one of these substituents not being H, in liquid ammonia at its boiling temperature in the presence of sodium amide to obtain a compound of the formula IV

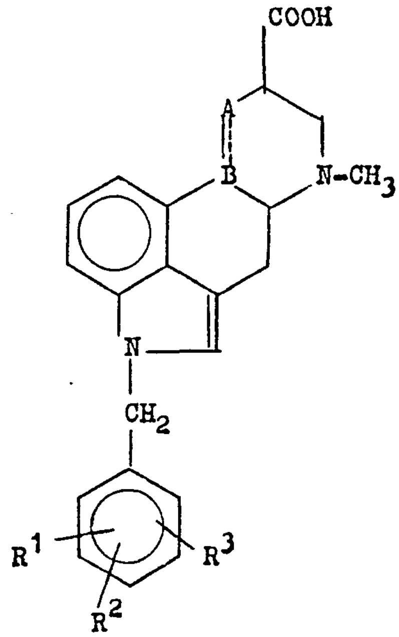

(IV)

wherein A ⋯ B and $R^1$, $R^2$ and $R^3$ are as defined above, and

b) reaction of the compound of the formula IV in an activated form with L-(+)-2-aminopropane-1-ol to obtain the corresponding compound of the formula I.

14. A process according to claim 12 or 13, characterized in that reaction products of the formula I wherein A ⋯ B is $-CH=C<$ are transformed into the corresponding 9.10-dihydroderivative by catalytic hydrogenation on Raney-nickel.

15. A process according to claim 13, characterized in that in step (b) the mixed anhydride of the compound of the formula IV with sulfuric acid is used as activated form.

16. A process according to claim 12 for preparing the compounds of the formula I wherein A ⋯ B is $-CH_2-CH<$, characterized in that a solution of the compound of the formula V in dimethylformamide is added dropwise at room temperature to a mixture of a compound of the formula II wherein A ⋯ B is defined as above and a pulverized alkali hydroxide in dimethylformamide.

17. A process according to one of claims 12 to 16, characterized in that the obtained bases are transformed with physiologically acceptable acids into the corresponding salts.

18. A process according to claim 17, characterized in that the maleinates, tartrates or methanesulfonates are prepared.

19. Pharmaceutical compositions, characterized by a 1-benzylated ergometrine derivative of the formula I according to one of claims 1 to 11 or one of their physiologically acceptable acid addition salts respectively, as active ingredients.

20. Anti-migraine therapeutics in the form of tablets, dragees, solutions for oral administration or injection solutions having a content of the active ingredient of 8,25 to 2,0 mg per dose unit.

**Revendications**

1. Dérivés de l'ergométrine 1-benzylés de la formule générale I

(Voir formule p. 14)

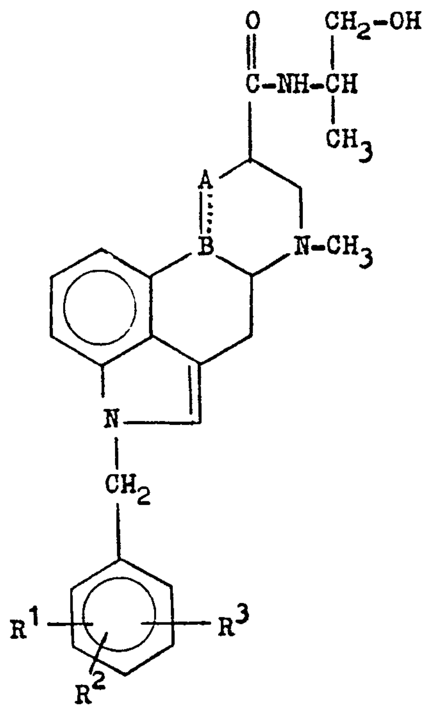

(I)

dans laquelle signifient :

$A \cdots B$ $-CH=C<$ ou $-CH_2-CH<$

et

$R^1$, $R^2$ et $R^3$ simultanément ou indépendamment hydrogène, halogène, alcoyle inférieur, trifluorométhyle ou alcoxy, au moins un des substituants $R^1$, $R^2$ et $R^3$ n'étant pas l'hydrogène, et leurs sels avec des acides physiologiquement acceptables.

2. Dérivés de l'ergométrine 1-benzylés de la formule I selon la revendication 1 sous forme des maléinates, tartrates ou méthanesulfonates.

3. 1-(2'-Fluorobenzyl)-ergométrine.

4. 1-(2'-Fluorobenzyl)-9.10-dihydroergométrine.

5. 1-(3'-Fluorobenzyl)-ergométrine.

6. 1-(3'-Fluorobenzyl)-9.10-dihydroergométrine.

7. 1-(4'-Fluorobenzyl)-ergométrine.

8. 1-(4'-Chlorobenzyl)-ergométrine.

9. 1-(4'-Chlorobenzyl)-9.10-dihydroergométrine.

10. 1-(2'-Bromobenzyl)-ergométrine.

11. 1-(2'-Méthylbenzyl)-ergométrine.

12. Procédé pour la préparation des dérivés de l'ergométrine 1-benzylés de la formule I selon l'une quelconque des revendications 1 à 11, caractérisé par réaction d'un dérivé de l'ergométrine de la formule générale II

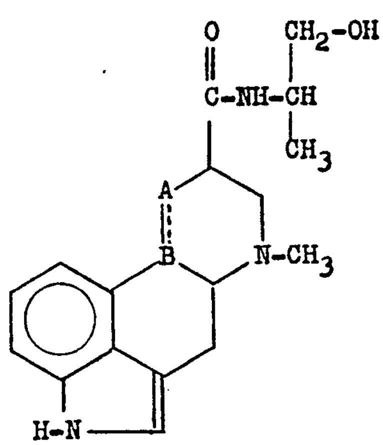

(II)

dans laquelle A⋯B a la signification —CH = C< ou —$CH_2$—CH<, avec une halogénure de benzyle substituée ayant la formule V,

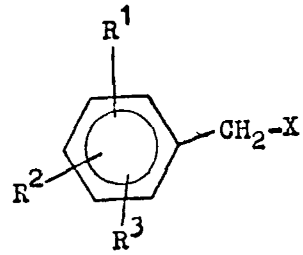

(V)

dans laquelle signifient :

X halogène

et

$R^1$, $R^2$ et $R^3$ simultanément ou indépendamment hydrogène, halogène, alcoyle inférieur, trifluorométhyle ou alcoxy, au moins un de ces substituants n'étant pas l'hydrogène, dans de l'ammoniaque liquide à la température de son ébullition en présence de l'amidure de sodium en obtenant le composé de la formule I correspondant.

13. Procédé pour la préparation des dérivés de l'ergométrine 1-benzylés de la formule I selon l'une quelconque des revendications 1 à 11, caractérisé par

a) réaction d'un acide lysergique de la formule générale III

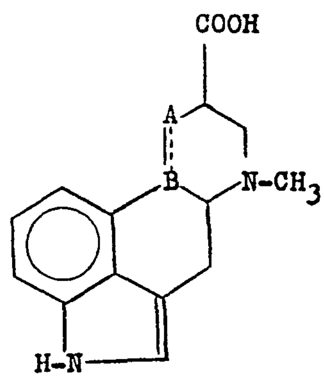

(III)

dans laquelle A⋯B a la signification —CH = C< ou —$CH_2$—CH<, avec une halogénure de benzyle substituée de la formule

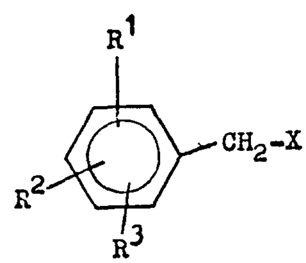

(V)

dans laquelle signifient :

X halogène

et

$R^1$, $R^2$ et $R^3$ simultanément ou indépendamment hydrogène, halogène, alcoyle inférieur, trifluorométhyle ou alcoxy, au moins un de ces substituants n'étant pas l'hydrogène, dans de l'ammoniaque liquide à la température de son ébullition en présence de l'amidure de sodium en obtenant un composé de la formule IV

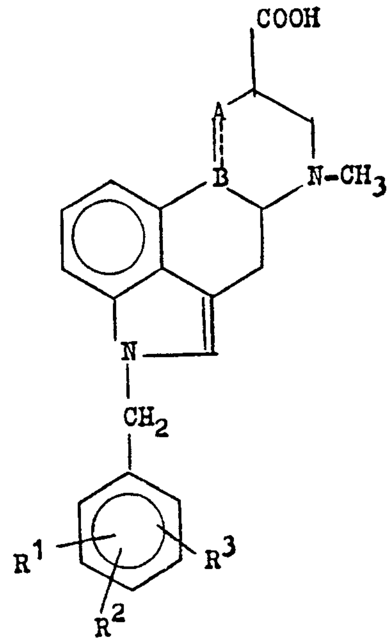

(IV)

A, B et $R^1$, $R^2$ et $R^3$ ayant la signification précitée,
et

b) réaction du composé de la formule IV dans une forme activée avec du L-(+)-2-aminopropan-1-ol en obtenant le composé correspondant de la formule I.

14. Procédé selon la revendication 12 ou 13, caractérisé en ce qu'on transforme des produits réactionnels de la formule I dans laquelle A ⋯ B a la signification de —CH = C< en le dérivé 9.10-dihydro correspondant par hydrogénation catalytique avec du nickel de Raney.

15. Procédé selon la revendication 13, caractérisé en ce qu'on utilise l'anhydride mixte du composé de la formule IV avec l'acide sulfurique comme forme activée dans l'étape (b).

16. Procédé selon la revendication 12 pour la préparation des composés de la formule I dans laquelle A ⋯ B a la signification de $—CH_2CH<$, caractérisé en ce qu'on ajoute, goutte à goutte, à la température ambiante une solution du composé de la formule V dans du diméthylformamide à un mélange d'un composé de la formule II dans laquelle A ⋯ B a la signification précitée et d'un hydroxyde alcalin pulvérisé dans du diméthylformamide.

17. Procédé selon l'une quelconque des revendications 12 à 16, caractérisé en ce qu'on transforme les bases obtenues en les sels correspondants avec des acides physiologiquement acceptables.

18. Procédé selon la revendication 17, caractérisé en ce qu'on produit les maléinates, les tartrates ou les méthanesulfonates.

19. Compositions pharmaceutiques, caractérisées par un dérivé de l'ergométrine 1-benzylé de la formule I selon l'une quelconque des revendications 1 à 11 ou bien d'un de leurs sels d'addition avec des acides physiologiquement acceptables comme matière active.

20. Compositions pour la thérapie de la migraine sous forme de comprimés, dragées, solutions pour l'administration orale ou solutions d'injections ayant une teneur en matière active de 0,25 à 2,0 mg par unité de dose.